# EUROPEAN PATENT SPECIFICATION

(11) **EP 3 073 889 B1**
(45) Date of publication and mention of the grant of the patent: **03.04.2019**
(21) Application number: 13811297.4
(22) Date of filing: 26.11.2013
(51) Int. Cl.: A61B 1/00, A61B 1/015, A61M 39/22

(54) **DISPOSABLE AIR/WATER VALVE FOR AN ENDOSCOPIC DEVICE**
EINWEG-LUFT/WASSERVENTIL FÜR EINE ENDOSKOPVORRICHTUNG
SOUPAPE AIR/EAU JETABLE POUR UN DISPOSITIF ENDOSCOPIQUE

(43) Date of publication of application: 05.10.2016
(73) Proprietor: United States Endoscopy Group, Inc., Mentor, OH 44060 (US)
(72) Inventor: BELLOFATTO, Steven, Closter, New Jersey 07624 (US); CUSHNER, Jeffrey B., Woodmere, New York 11598 (US); JOACHIM, Robert, Glen Rock, New Jersey 07452 (US); SALMON, Scott, Tenafly, New Jersey 07670 (US); WOLCOTT, Kenneth E., Centerport, New York 11721 (US)
(74) Representative: Petraz, Gilberto Luigi
(86) International application number: PCT/US2013/071871
(87) International publication number: WO 2015/080694

(56) References cited:
- EP-A1- 1 741 460
- WO-A1-2012/075131
- WO-A2-2010/111546
- US-A1- 2004 238 014

## Description

### FIELD OF THE INVENTION

The present invention relates generally to air/water valves for use with medical instruments. More particularly the present invention relates to a disposable valve assembly for use in an endoscopic device.

### BACKGROUND OF THE INVENTION

Endoscopes are used in modern medical practices to allow a medical practitioner to look inside a hollow organ or body cavity of a patient. Using an endoscope, a patient's symptoms may be investigated (e.g., symptoms in the digestive system including nausea, vomiting, abdominal pain, difficulty swallowing, gastrointestinal bleeding, etc.); a diagnosis may be confirmed (e.g., by performing a biopsy); or treatment may be provided (e.g., cauterizing a bleeding vessel, widening a narrow esophagus, clipping off a polyp, etc.).

Unlike other medical imaging devices, endoscopes are inserted directly into the organ or cavity. During an endoscopic procedure, air and water are typically used to insufflate the organ or cavity being accessed and/or to irrigate the area and/or portions of the device (e.g., the optic head of the endoscope itself). The flow of air and water is typically controlled by the user of the device via a valve.

WO 2012/075131 discloses a disposable air/water valve that comprises a main stem having a proximal end, the main stem comprising a plurality of ridges and grooves disposed circumferentially about the main stem. The plurality of ridges and grooves is monolithic with the main stem. The main stem comprises a first opening disposed at the
proximal end and running along a longitudinal axis of the main stem, and a second opening disposed transverse to the first opening.

EP 1741460 discloses an inhalation device that comprises a control means that keeps the inhalation flow essentially constant during the entire aerosol inhalation. The control means comprises a controllable valve having a housing, a closure element, and a membrane. The valve comprises an optional pressure plate, a set piston and an adjusting screw for adjusting the flow rate through the controllable valve.

US 2004/238014 discloses a fixture for holding a valve assembly in an automated washing system. The valve assembly is movable between an opened position and a normally closed position. The fixture is comprised of first and second housing sections
that are releaseably attachable to each other. The housing sections define an internal cavity that is dimensioned to hold the valve assembly in an opened position. A fluid inlet is in communication with the chamber, and is attachable o a source of an anti-microbial fluid in the washing system. A fluid outlet communicates with the chamber to allow the anti-microbial fluid to exit the fixture.

WO 2010/111546 discloses a medical connector for use in a fluid pathway. The medical connector has a backflow resistance module configured to prevent fluid from being drawn into the connector when a backflow inducing event occurs. The backflow resistance module can include a variable-volume chamber configured to change in volume in response to a backflow-inducing event and a check valve configured to resist backflow.

The medical connector can include a fluid diverter configured to direct fluid flowing through the medical connector into the variable volume chamber to prevent fluid stagnation therein. The medical connector includes a body member, a base member, a seal member, a support member, and a valve member.

### SUMMARY OF THE INVENTION

Valves such as those described above for regulating the flow of air and water through an endoscope must be cleaned and disinfected after every medical procedure to avoid cross-contamination between patients. This necessitates having several valves on hand to accommodate back-to-back procedures, as the cleaning and disinfection processes can take significant time to complete. Reusable valve are also expensive and must be handled with care.

Accordingly, embodiments of the present invention described herein provide for a disposable valve assembly for regulating the flow of air and water through a medical instrument, such as an endoscope. Embodiments of the valve assembly described herein are designed for use in a single procedure and can then be discarded.

As described below, in one embodiment, a valve assembly for a medical instrument is provided that includes a housing and a spool comprising a first end, a second end, a longitudinal passageway extending between the first end and the second end, and first and second portions proximate the first and second ends, respectively. The first portion of the spool may be configured to be received at least partially within the housing, and an outer surface of the second portion of the spool may define a plurality of positioning features. The valve assembly may further include a sealing member comprising a longitudinal support member and a plurality of sealing rings extending circumferentially from the support member. Each sealing ring may be spaced from an adjacent sealing ring a distance along a length of the support member that corresponds to a distance between adjacent positioning features. An inner surface of the support member may be configured to engage the outer surface of the spool. Furthermore, each sealing ring may define an opening such that an inner circumferential surface of each sealing ring is configured to sealingly engage the outer surface of the second portion of the spool proximate a corresponding positioning feature.

In some cases, the valve assembly may further include a spring and a retainer. The spring may comprise a first end and a second end and may be disposed around the first portion of the spool. The retainer may be configured to be received by the first end of the spool. The second end of the spring may be configured to engage and remain fixed with respect to the housing, and the first end of the spring may be configured to engage the retainer, such that the retainer is biased away from the housing. The housing may define at least one radial extension configured to engage the second end of the spring. At least a portion of the outer surface of the spool may define a longitudinal groove configured to receive the support member of the sealing member. An outer surface of the support member may form a flush surface with the outer surface of the spool when the sealing member is engaged with the spool. Furthermore, the sealing member may be overmolded onto the spool.

In some embodiments, the spool may define a transverse passageway that is substantially perpendicular and intersects with the longitudinal passageway. The transverse passageway may be defined through the longitudinal groove. At least one portion of the longitudinal groove may be defined by at least one positioning feature. In addition, the spool may define a transverse passageway that is substantially perpendicular to and intersects with the longitudinal passageway, and the support member of the sealing member may define a longitudinally extending ring configured to engage an opening of the transverse passageway defined in the outer surface of the spool.

The sealing member may comprise four sealing rings in some cases. In addition, the housing may define longitudinal extensions that are configured to engage an endoscope within which the valve assembly is mounted.

In other embodiments, a method of manufacturing a valve assembly for a medical instrument is provided. The method may include molding a spool, where the spool comprises a first end, a second end, a longitudinal passageway extending between the first end and the second end, and first and second portions proximate the first and second ends, respectively. An outer surface of the second portion of the spool may define a plurality of positioning features.

The method may further include overmolding a sealing member onto an outer surface of the spool. The sealing member may comprise a longitudinal support member and a plurality of sealing rings extending circumferentially from the support member. Each sealing ring may be spaced from an adjacent sealing ring a distance along a length of the support member that corresponds to a distance between adjacent positioning features. An inner surface of the support member may engage the outer surface of the spool, and each sealing ring may define an opening such that an inner circumferential surface of each sealing ring may sealingly engage the outer surface of the second portion of the spool proximate a corresponding positioning feature. Furthermore, a housing may be molded that is configured to at least partially receive the first portion of the spool, and the first portion of the spool may be disposed at least partially within the housing.

In some cases, the method may include disposing a spring around the first portion of the spool, wherein the spring defines a first end and a second end, and providing a retainer configured to be received by the first end of the spool. The second end of the spring may be engaged to the housing such that a position of the second end of the spring remains fixed with respect to the housing, and the first end of the spring may be engaged with the retainer. The retainer may be attached to the first end of the spool, such that the retainer is biased away from the housing.

In some embodiments, a longitudinal groove may be defined in at least a portion of the outer surface of the spool, wherein the longitudinal groove is configured to receive the support member of the sealing member. Molding the spool may comprise using a first mold to form the spool, and overmolding the sealing member may comprise removing the spool from the first mold and placing the spool in a second mold. Overmolding the sealing member may comprise directing an elastomeric material into the longitudinal groove to form the sealing member in the second mold.

In some cases, a plurality of stationary plates may be arranged in an orientation that is perpendicular to a longitudinal axis of the spool. The plates may comprise concentric holes and may be positioned at locations corresponding to locations of the sealing rings to be formed. Moreover, molding the spool may comprise defining a transverse passageway that is substantially perpendicular to and intersects with the longitudinal passageway, and wherein the transverse passageway is defined through the longitudinal groove. At least one portion of the longitudinal groove may be defined by at least one positioning feature. Additionally or alternatively, molding the spool may comprise defining a transverse passageway that is substantially perpendicular and intersects with the longitudinal passageway, and overmolding the sealing member may comprise defining a longitudinally extending ring in the support member that engages an opening of the transverse passageway defined in the outer surface of the spool.

Such embodiments provide significant advantages as described and otherwise discussed herein.

### BRIEF DESCRIPTION OF THE DRAWINGS

Reference will now be made to the accompanying drawings, which are not necessarily drawn to scale. The drawings are for illustrative purposes only, and are not intended to limit the scope of the present invention.
**FIG. 1** shows a schematic illustration of an endoscopic device;
**FIG. 2A** shows a cross-sectional view of a conventional air/water valve assembly in an unactuated position;
**FIG. 2B** shows a cross-sectional view of the air/water valve assembly of FIG. 2A in an actuated position;
**FIG. 3** shows a perspective view of a conventional air/water valve assembly;
**FIG. 4** shows an exploded view of the air/water valve assembly of FIG. 3;
**FIG. 5** shows a perspective view of an air/water valve assembly according to an exemplary embodiment of the present invention;
**FIG. 6** shows an exploded view of the air/water valve assembly of FIG. 5 according to an exemplary embodiment of the present invention;
**FIG. 7A** shows a perspective view of a spool according to an exemplary embodiment of the present invention;
**FIG. 7B** shows a cross-sectional view of the spool of FIG. 7A;
**FIG. 8** shows a perspective view of a one-piece sealing member according to an exemplary embodiment of the present invention;
**FIG. 9** shows a perspective view of a housing according to an exemplary embodiment of the present invention;
**FIG. 10** shows a view of a valve assembly inserted within an air/water cylinder of a medical device according to an exemplary embodiment of the present invention;
**FIG. 11** illustrates a flowchart of a method for manufacturing an air/water valve assembly according to an exemplary embodiment of the present invention; and
**FIGs. 12A** and **12B** show schematic representations of a mold for manufacturing an air/water valve assembly according to an exemplary embodiment of the present invention.

### DETAILED DESCRIPTION OF THE INVENTION

Embodiments of the present invention will be described with reference to the accompanying drawings, where applicable. It is understood that the present invention may be embodied in many different forms and should not be construed as limited to the embodiments set forth herein; rather, these embodiments are provided for illustrative purposes only. Like numbers refer to like elements throughout.

While the embodiments of the valve assembly and method for making the valve assembly for regulating the flow of air and water through a medical instrument are described below in the context of an endoscope for performing an endoscopic procedure (such as a colonoscopy), it should be understood that the embodiments of the present invention may also be utilized in other medical instruments including, for example, a variety of different endoscopic and/or laparoscopic instruments.

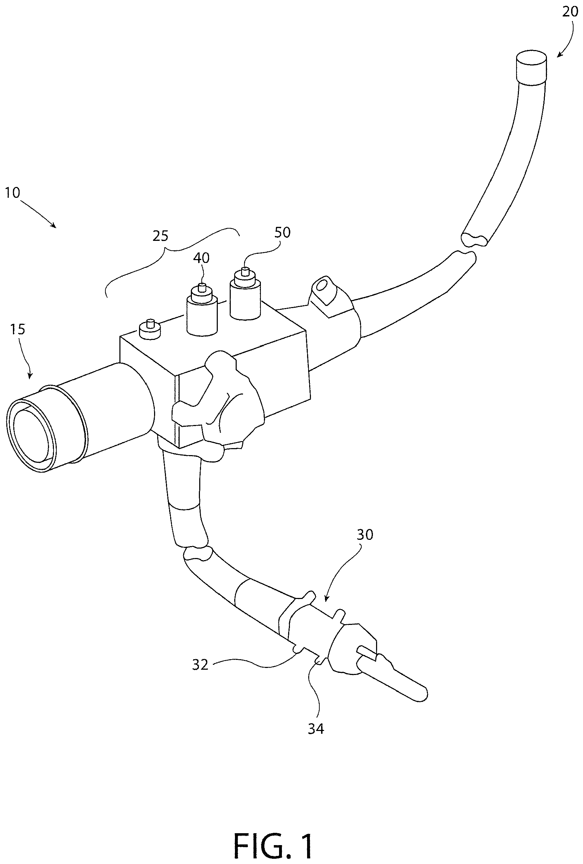

An example of an endoscope 10 for performing endoscopic procedures, such as a gastrointestinal endoscopy, is shown in Fig. 1. The depicted endoscope 10 includes a proximal end 15, which may be the end closest to the user (e.g., the medical practitioner) and may include an eyepiece that the user can look through to view the organ or cavity being examined. The endoscope 10 also includes a distal end 20, which may be the end closest to the target site within the organ or cavity being examined. Controls may be provided on a main body 25 of the endoscope disposed between the proximal and distal ends 15, 20. Furthermore, a fluid conducting portion 30 may be provided that connects a source of air and water (not shown) with the distal end 20 of the endoscope 10. For example, air, CO₂, or other gas for distending the organ or cavity is able to enter the endoscope 10 via a gas inlet 32, and water is able to enter via a water inlet 34. The flow of either fluid towards the distal end 20 may be regulated and controlled via the user's interaction with the controls of the main body 25. The main body 25 of the endoscope 10 may house a suction valve 40 for withdrawing effluent and gases from the gastrointestinal tract, as well as an air/water valve 50 for controlling the flow of air and/or water through the endoscope.

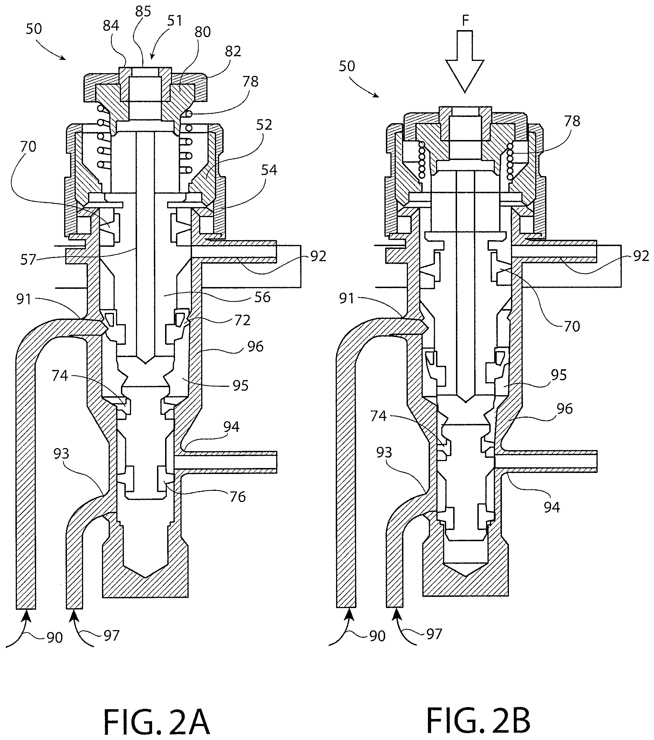

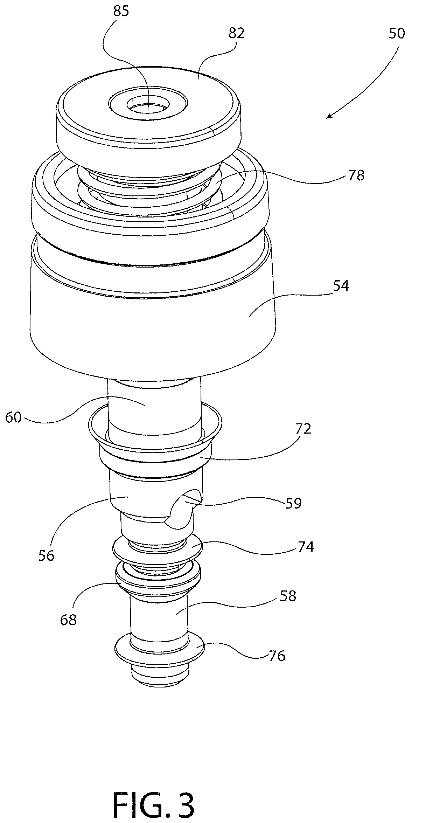

The operation of the air/water valve 50 is shown in the cross-sectional views of Figs. 2A and 2B. For example, an unactuated position of the valve 50 is shown in Fig. 2A, and an actuated position of the valve is shown in Fig. 2B. As illustrated in Fig. 2A, a spring 78 may be provided that engages a spring retainer 80 at one end and a valve housing 52, 54 at the other end. The spring retainer 80 may be attached to the spool 56 (e.g., via other valve components shown in Fig. 3), such that the spring 78 biases the spool 56 to the unactuated position shown in Fig. 2A. At the same time, an actuation force F applied to the outer end 51 of the valve 50 by the user (as shown in Fig. 2B) may serve to move the spool 56 and its components with respect to the housing 52, 54 to the actuated position of Fig. 2B.

As shown in Figs. 2A and 2B, at least a portion of the spool 56 is configured to define a passageway 57 therethrough. The spool 56 may, for example, define a port 59 that is oriented perpendicularly with respect to the longitudinal axis of the passageway 57 of the spool 56, and the passageway 57 may extend from the port 59 to an opening 85 at the outer end 51 of the valve 50.

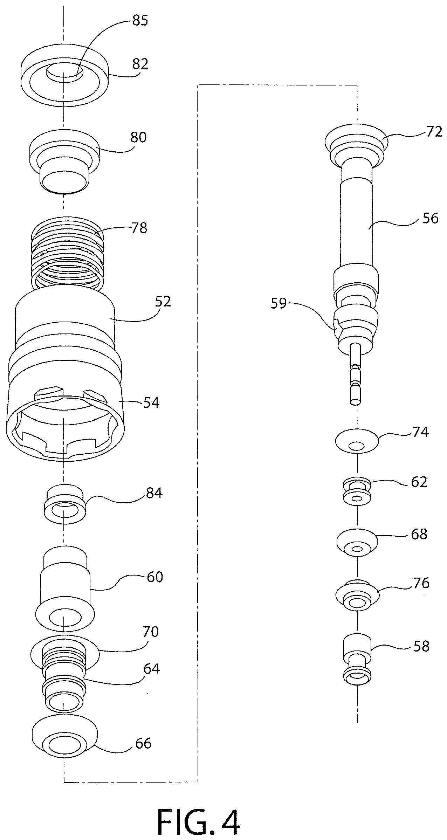

With reference to Fig. 2A, air 90 (or other gas) may be able to flow from the gas inlet 32 (shown in Fig. 1) into a cavity 95 defined by an air/water cylinder 96 in which the valve 50 is disposed via an inlet 91. When the opening 85 is clear (e.g., when the user is not blocking the opening 85 with a finger, for example), the air 90 may flow from the cavity 95, through the port 59, through the passageway 57, and out of the valve assembly via the opening 85. When the opening 85 is blocked by the user's finger, for example, pressure within the passageway 57 may build up to a point at which the air 90 is forced past a lip seal 72 that is positioned around the spool 56 (e.g., by deflecting the seal away from the inner surface of the air/water cylinder 96, towards the spool 56) and out of the cavity 95 past the bearing 70 towards an outlet in the distal end 20 of the endoscope via an outlet 92. At the same time, the flow of water 97 from an inlet 93 is precluded from exiting the cavity 95 via an outlet 94, as the seal 76 (shown also in Fig. 4) does not allow the air to flow past.

When the user applies a force F against the biasing force of the spring 78 to actuate the valve 50, the valve is moved to the actuated position shown in Fig. 2B. In the actuated position, air 90 is precluded from exiting the cavity 95 via outlet 92 as a result of the movement of a seal 70 to a position shown in Fig. 2B that blocks the air from accessing the outlet 92. In addition, the seal 76 that was blocking the passage of water from the inlet 93 to the outlet 94 of the air/water cylinder 96 is also moved so as to clear a pathway for the flow of water to the outlet 94. In the actuated position shown in Fig. 2B, the seal 74 blocks the water from the inlet 92 from passing into the space in the cavity 95 through which the airflow path from the inlet 91 to the outlet 92 is defined.

With reference now to Figs. 3 and 4, a conventional reusable air/water valve 50, as described above, is shown in assembled and exploded configurations. Conventional reusable valves 50 typically include a number of machined metal parts that, along with multiple seals, must be assembled together to form a valve that is able to function as described above with respect to Figs. 2A and 2B. For example, the depicted valve (shown in exploded form in Fig. 4) includes a housing 52 and a housing overmold 54; a spool 56 with spool ends 58, 60 and spool sleeves; two bearings 66, 68; four seals 70, 72, 74, 76; a spring 78, spring retainer 80, and retainer cap 82; and a color ring 84 for a total of seventeen separate components. Of these components, six are machined metal components (e.g., the housing 52, the spool 56, the spool ends 58, 60, and the spool sleeves 62, 64). The large number of components results in a complex design that is costly to fabricate and laborious to assemble. Moreover, the reusable nature of the valve 50 requires the valve to be cleaned and disinfected after every use to prevent cross-contamination between patients, which also adds to the cost.

Accordingly embodiments of the present invention provide an air/water valve that is made up of fewer components than conventional reusable valves such as the example described above and shown in Figs. 2A-4. Moreover, in embodiments of the present invention, all of the components can be produced using injection molding, thereby eliminating the need for machined metal parts. In this way, embodiments of the present invention provide a valve that can be used as a disposable (e.g., single-use) valve in a cost-effective manner.

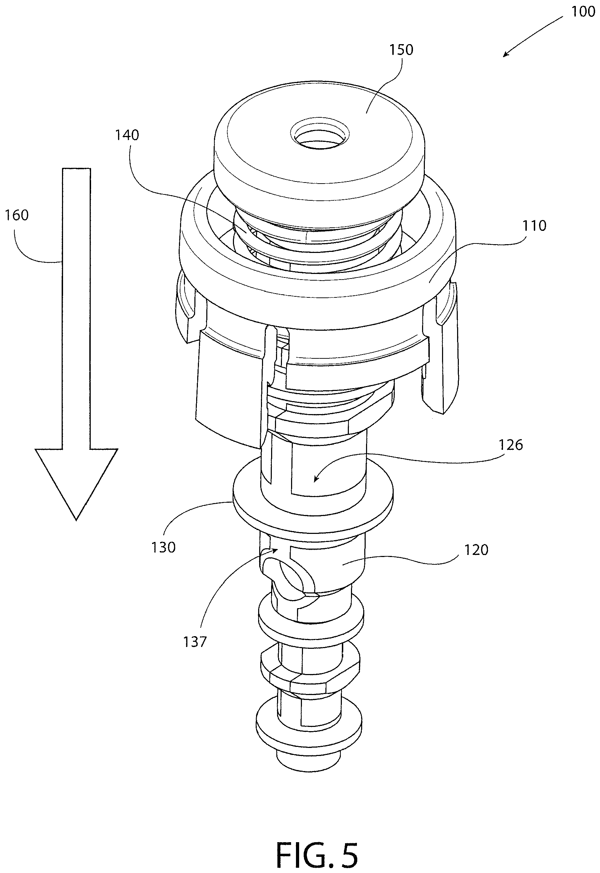

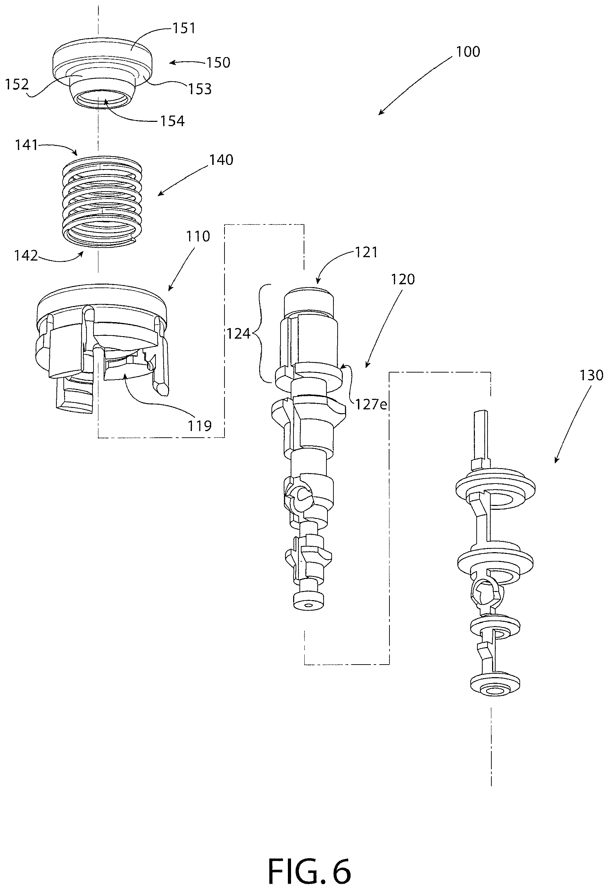

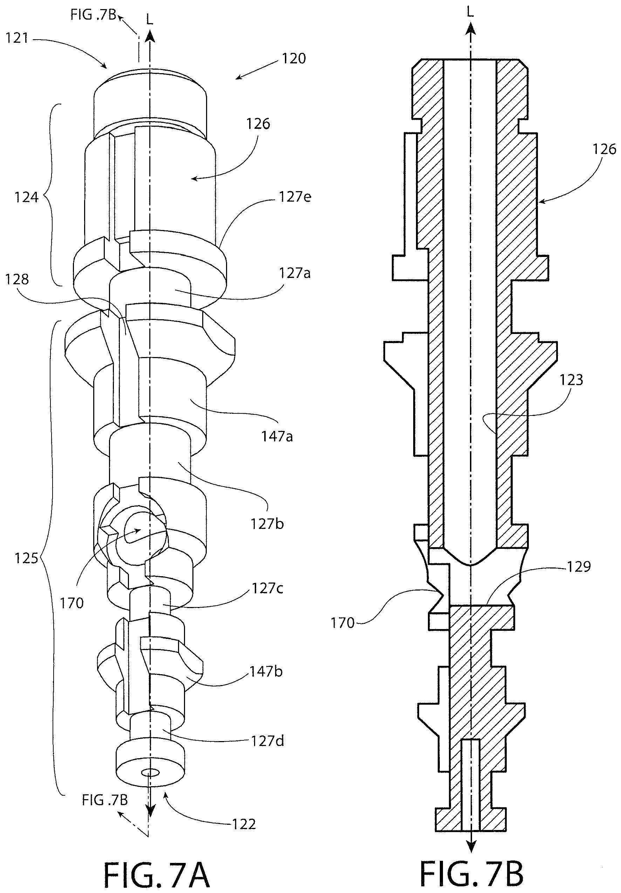

Turning now to Figs. 5 and 6, a valve assembly 100 is shown in accordance with embodiments of the present invention. In the depicted embodiment, the valve assembly 100 includes a housing 110, a spool 120, and a sealing member 130. With reference to Figs. 7A and 7B, the spool 120 includes a first end 121, a second end 122, and a longitudinal passageway 123 extending between the first end and the second end. The spool 120 further comprises a first portion 124 proximate the first end 121 and a second portion 125 proximate the second end 122. The first portion 124 of the spool 120 may be configured to be received at least partially within the housing 110.

Moreover, an outer surface 126 of the spool 120 may define a plurality of bearing features 147a, 147b and/or a plurality of positioning features 127a, 127b, 127c, 127d. As depicted, the bearing features 147a, 147b and the positioning features 127a, 127b, 127c, 127d may provide the spool 120 with a surface profile such that the diameter of the spool varies along the spool's longitudinal axis *L*.

The bearing features 147a, 147b may, for example, comprise portions of the spool that bulge or otherwise protrude radially outwardly from the surface 126 of the spool 120, such that they provide regions of the spool that have a larger diameter than other regions of the spool (e.g., a larger diameter than adjacent regions). In this regard, the bearing features 147a, 147b may be configured (e.g., sized, shaped, and/or positioned) to serve a function similar to the bearings 66, 68 of the conventional valve shown in Fig. 4.

The positioning features 127a, 127b, 127c, 127d may, for example, comprise ridges, concavities, and/or other features that are configured to receive complementary portions of the sealing member 130, as described in greater detail below. For example, the positioning features 127a, 127b, 127c, 127d may be configured such that the material for forming the sealing rings 132, 133, 134, 135 of the sealing member may be injection molded into the positioning features.

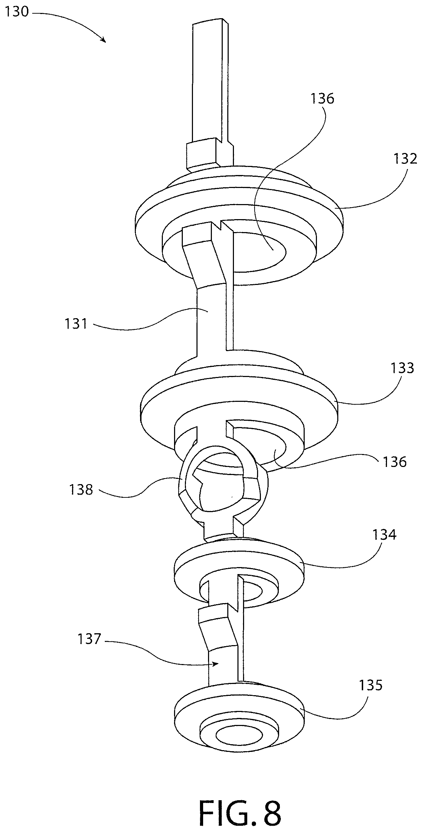

A close-up view of the sealing member 130 is shown in Fig. 8. The sealing member 130 may include a longitudinal support member 131 and a plurality of sealing rings extending circumferentially from the support member 131. In the depicted embodiment, the sealing member 130 includes four sealing rings 132, 133, 134, 135. Each sealing ring 132, 133, 134, 135 may, for example, be spaced from an adjacent sealing ring by a distance along a length of the support member 131 that corresponds to a distance between adjacent positioning features 127a, 127b, 127c, 127d. For example, referring to Figs. 7A and 8, the positioning features 127a and 127b in Fig. 7A may correspond to the distance between sealing ring 132 and 133 in Fig. 8. Likewise, the distance between the positioning features 127b and 127c may correspond to the distance between the sealing rings 133 and 134, and the distance between the positioning features 127c and 127d may correspond to the distance between the sealing rings 134 and 135.

Accordingly, an inner surface 136 of the support member 131 may be configured to engage the outer surface 126 of the spool 120. For example, each sealing ring 132, 133, 134, and 135 may define an opening therethrough, such that an inner circumferential surface 136 of each sealing ring may be configured to sealingly engage the outer surface 126 of the second portion 125 of the spool 120 proximate a corresponding positioning feature 127a, 127b, 127c, 127d. In this way, the sealing rings 132, 133, 134, and 135 may be configured to serve as the seals 70, 72, 74, 76 of the conventional valve shown in Fig. 4 when assembled as a valve assembly and disposed within an air/water cylinder (such as the air/water cylinder 96 shown in Figs. 2A and 2B). In contrast with the seals in a conventional valve, such as the valve 50 of Figs. 2A and 2B, embodiments of the invention provide sealing rings 132, 133, 134, 135 that consist of only 1 piece of injection molded plastic that is, in some embodiments, overmolded directly onto the spool 120 in the configuration shown in Fig. 5, as described in greater detail below.

Turning again to Fig. 6, in some embodiments, the valve assembly 100 may further include a spring 140 and a retainer 150. Although a coil spring is depicted in Fig. 6, other types of spring structures and materials may be used in other embodiments to provide the same biasing forces. Thus, the spring 140 may be a coil spring (metal or plastic), a spring washer-type spring (metal or plastic), or a cylinder of foam material.

The spring 140 may have a first end 141 and a second end 142 and may be disposed around the first portion 124 of the spool 120, as shown in Figs. 5 and 6. The retainer 150, in turn, may be configured (e.g., sized and shaped) to be received within the first end 141 of the spring 140 and to engage the first end 121 of the spool 120. For example, the retainer 150 may include a cap portion 151 and a reduced-diameter portion 152 extending from the cap portion. A lip 153 may be defined by the cap portion 151 proximate the interface between the cap portion and the reduced-diameter portion 152. The reduced-diameter portion 152 may be configured to fit inside the spring 140 (e.g., inside the coils of the spring in the depicted embodiment), and the first end 141 of the spring 140 may thus be configured to engage (e.g., contact and/or push against) the lip 153 when the valve assembly is assembled. Moreover, the reduced-diameter portion 152 may define an opening 154 therethrough that is configured to receive and engage at least part of the first portion 124 of the spool 120. For example, the spool 120 may be attached to the retainer 150 via a press fit engagement between the first end 121 of the spool and the opening 154 within which it is received. Additionally or alternatively, adhesive may be used to secure the first end 121 of the spool 120 within the opening 154 of the retainer 150. In such a way, the retainer 150 may be fixed with respect to the spool 120, and the spring 140 may be retained in a surrounding relationship with respect to the first portion 124 of the spool 120.

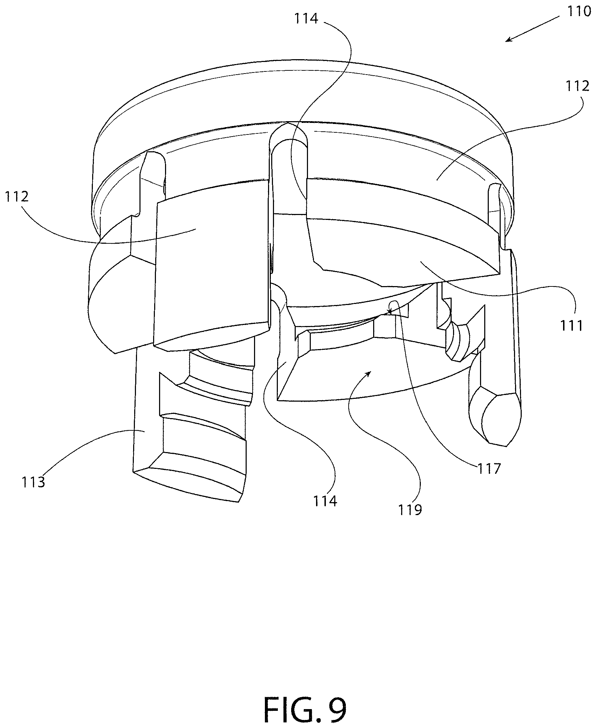

At the same time, the second end 142 of the spring 140 may be configured to engage and remain fixed with respect to the housing 110. For example, as shown in Fig. 9, in some embodiments, the housing 110 may define radial extensions 111 that extend inwardly from an outer wall 112 of the housing. The second end 142 of the spring 140 may, in such embodiments, engage and push against an inner surface 117 of the radial extensions 111 of the housing 110, shown in Fig. 9, such that an actuating force applied to the spring 140 (such as by pushing on the retainer 150 in the direction of the spool 120 in the assembled configuration shown in Fig. 5) is resisted by the radial extensions 111 and the spring 140 remains between the lip 153 of the retainer and the inner surface 117 of the radial extensions 111 of the housing. In the unactuated position shown in Fig. 5, the retainer 150 may be biased away from the housing 110. The retainer 150 and spool 120 assembly is maintained in engagement with the housing 110, however, through the engagement of a spool ledge 127e with an outer surface 119 of the radial extensions 11 of the housing 110 (shown in Figs. 6, 7A, and 9).

Thus, with the second end 142 of the spring 140 engaged with the housing 110, the first end 141 of the spring engaged with the retainer 150, and the retainer engaged with the first end 121 of the spool, as described above, an actuating force applied to the retainer 150 when the valve assembly 100 is in the unactuated position shown in Fig. 5 serves to move the spool 120 and the sealing member 130 attached thereto in the direction indicated in Fig. 5 by the arrow 160. When the actuating force is removed, the biasing force of the spring 140 moves the spool 120 and sealing member 130 back towards the unactuated position depicted in Fig. 5. Accordingly, movement of the spool 120 and the sealing member 130 within an air/water cylinder such as the cylinder 96 shown in Figs. 2A and 2B within an endoscope or other medical instrument may open and close certain air and water pathways, similarly to the function of the valve 50 described above with respect to Figs. 2A and 2B.

Turning again now to Fig. 7A, in some embodiments, at least a portion of the outer surface 126 of the spool 120 may define a longitudinal groove 128 that is configured to receive the support member 131 of the sealing member 130 (shown in Fig. 8), as shown in the assembled configuration depicted in Fig. 5. The longitudinal groove 128 and the support member 131 may be configured, for example, such that the outer surface 137 of the support member 131 forms a flush surface with the outer surface 126 of the spool 120 when the sealing member 130 is engaged with the spool. As noted above, the tight fit of the sealing member 130 with the spool 120 (e.g., within the longitudinal groove 128) may, for example, be the result of the overmolding of the sealing member onto the spool, as described in greater detail below. In other cases, however, the sealing member 130 may be formed (e.g., molded) separately from the spool 120 and may be configured (e.g., through sizing and material selection) such that it can be stretched to fit onto the spool 120.

As shown in the cross-section of Fig. 7B, in some cases the spool 120 may define a transverse passageway 129 that is substantially perpendicular to and intersects with the longitudinal passageway 123. The transverse passageway 129 may be defined through the longitudinal passageway 123, so as to provide a flow path for the air or other gas to enter into the longitudinal passageway and be directed towards the first end 121 of the spool 120. The air or other gas may then be released to the external environment via opening 154 in the cap portion 152. In this regard, the transverse passageway may be defined through the longitudinal groove 128.

In still other embodiments, at least one portion of the longitudinal groove 128 may be defined by at least one positioning feature, as described above. For example, in the depicted embodiment of Fig. 7A, the longitudinal groove 128 extends through and is defined by the bearing features 147a, 147b and/or the positioning features 127a, 127b, 127c, 127d. Moreover, in some cases, such as when the transverse passageway 129 is defined through the longitudinal groove 128, the support member 131 of the sealing member 130 may define a longitudinally extending ring 138 that is configured to engage the opening 170 of the transverse passageway 129 defined in the outer surface 126 of the spool 120, as shown in Fig. 5. In other cases, however, such as when the transverse passageway 129 is not defined through the longitudinal groove 128 (e.g., when the location of the longitudinal groove is rotated 90° about the longitudinal axis L from the position depicted in Fig. 7A), the support member 131 of the sealing member 130 need not define a longitudinally extending ring 138 and may, instead, extend linearly from one end of the sealing member to the other.

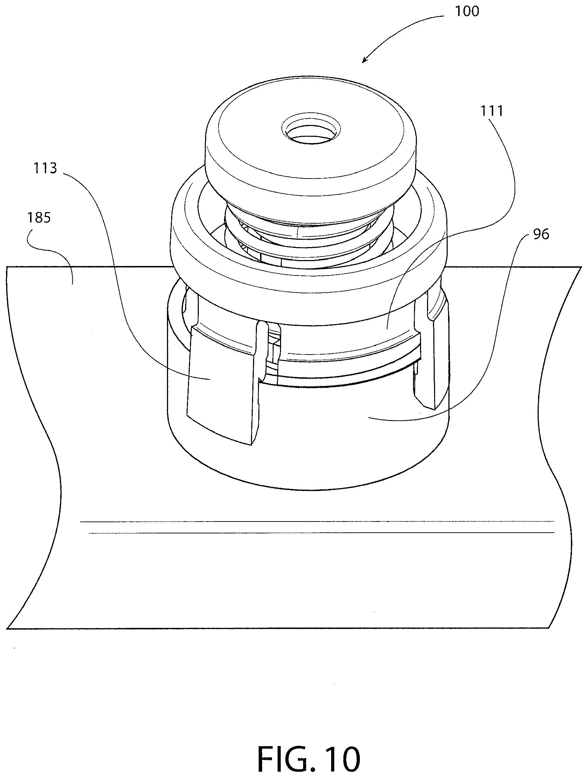

With reference to Fig. 9, in some embodiments, the housing 110 defines at least one longitudinal extension 113 configured to extend over the outer surface 126 of the first portion of the spool 120 or at least a portion thereof, as shown in Fig. 5. In the depicted embodiment, for example, three longitudinal extensions 113 are provided, arranged in alternating fashion with portions of the outer wall 112 of the housing 110 that define three radial extensions 111. The longitudinal extensions 113 may be configured (e.g., sized and shaped) to secure the valve assembly 100 to an air/water cylinder of the endoscope or other medical device when the valve assembly is installed for use. With reference to Fig. 10, for example, the air/water cylinder 96 may extend upwardly from the body 185 of the endoscope or medical device, and the valve assembly 100 shown in Fig. 5 may be inserted into the air/water cylinder. As the valve assembly 100 is inserted into the air/water cylinder 96, the longitudinal extensions 113 may receive the air/water cylinder 96, and a top edge of the air/water cylinder may rest against the outer surface 119 of the radial extensions 111 of the housing 110 shown in Fig. 9 once the valve assembly 100 is fully engaged within the air/water cylinder. Accordingly, once the valve assembly 100 is inserted into the air/water cylinder 96, as depicted in Fig. 10, the longitudinal extensions 113 may form a snug fit with the outer surface of the air/water cylinder 96, thereby maintaining the valve assembly in engagement with the endoscope or other medical instrument during operation.

In some embodiments, slots 114 may be defined in the outer wall 112 of the housing 110. The slots 114 may be configured to facilitate engagement of the housing 110 with the air/water cylinder 96 by allowing the housing 110 of the valve assembly to form a positive lock with the endoscope or other medical instrument within which the valve assembly 100 is mounted. The slots 114, for example, may allow the longitudinal extensions 113 to act as tangs that are configured to be moved outwardly from the longitudinal axis of the valve assembly (e.g., by flexing) as the inner surface of the longitudinal extensions are guided over mating ridges defined by the outer surface of the air/water cylinder 96 as the valve assembly is inserted into an operating position therein. In other cases, however, the radial extensions 111 and the longitudinal extensions 113 may form a continuous outer wall 112 of the housing.

In some embodiments, one or more of the longitudinal extensions 113 may include a pull tab (not shown) on the outer surface of the respective longitudinal extension that can be grasped by a user and used to break off at least one of the longitudinal extensions once use of the valve assembly is compete. In this way, the valve assembly may be more easily removed from the endoscope after a single use (e.g., by "unlocking" the attachment of the valve assembly to the air/water cylinder). At the same time, the breaking of a longitudinal extension may prevent the use of the valve assembly in another endoscope for additional procedures, thereby ensuring that the valve assembly is a single-use valve assembly.

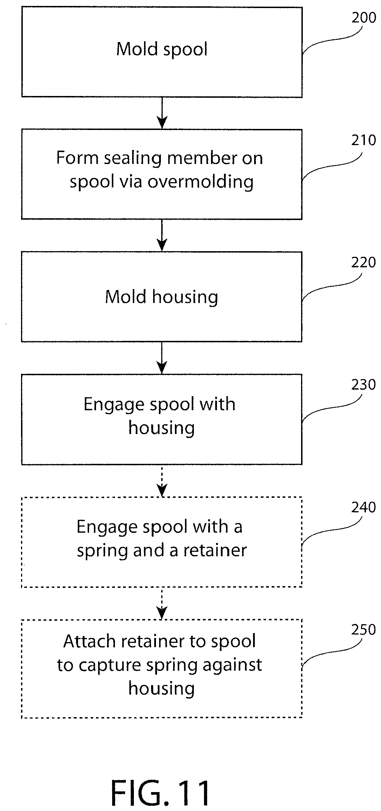

Turning now to Fig. 11, a method of making a valve assembly, such as the valve assembly 100 described above and illustrated in Figs. 5-9, is shown. According to embodiments of the method, a spool is molded at block 200. As described above, the spool may include a first end, a second end, a longitudinal passageway extending between the first end and the second end, and first and second portions proximate the first and second ends, respectively, where an outer surface of the second portion of the spool defines a plurality of positioning features. The spool may be molded in a conventional mold. Suitable materials for molding the spool may include, for example, polycarbonate (PC); acrylonitrile butadiene styrene (ABS); polypropylene; polyethylene; and other suitable polymers.

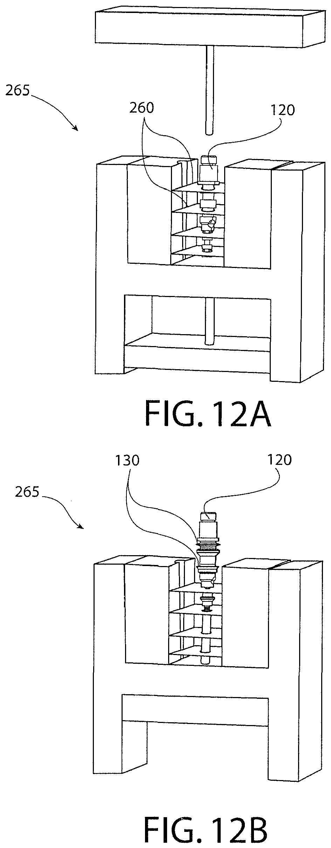

The spool may be removed from the first mold and placed in a second mold for overmolding the sealing member, as described at block 210. An illustration of an embodiment of the second mold 265 is shown in Figs. 12A and 12B. The sealing member may be made of elastomeric materials and may include a longitudinal support member and a plurality of sealing rings extending circumferentially from the support member, as described above. Each sealing ring may be spaced from an adjacent sealing ring a distance along a length of the support member that corresponds to a distance between adjacent positioning features defined by the spool, such that an inner surface of the support member engages the outer surface of the spool. Each sealing ring may thus define an opening such that an inner circumferential surface of each sealing ring sealingly engages the outer surface of the second portion of the spool proximate a corresponding adjacent positioning feature. In some embodiments, stationary plates defining concentric holes may be arranged in a spaced apart manner along the longitudinal axis of the spool. The stationary plates 260 may be arranged in an orientation that is perpendicular to the longitudinal axis of the spool, and a portion of the spool 120 may be received within the hole defined in each plate at a location at which a sealing ring is to be formed, as shown in Fig. 12A. In this way, the stationary plates may allow seal contact surfaces (e.g., an outer circumferential surface) of the sealing rings to be formed during the overmolding process without the formation of parting lines, which may otherwise cause leaks. The completed spool 120 with overmolded sealing member 130 may then be removed from the mold 265 shown in Fig. 12A by ejecting the spool and sealing member through the stationary plates 260, as shown in Fig. 12B.

In some embodiments, molding the spool may include defining a transverse passageway that is substantially perpendicular to and intersects with the longitudinal passageway of the spool, as described above. The transverse passageway may be defined through the longitudinal groove in some cases. Moreover, in some instances, overmolding the sealing member may comprise defining a longitudinally extending ring in the support member that engages an opening of the transverse passageway defined in the outer surface of the spool.

At block 220, a housing may be molded, where the housing is configured to at least partially receive the first portion of the spool. The housing may be made of any suitable material, such as a thermoplastic or polyester-based elastomer (e.g., rubber, Viton® material, silicone, neoprene, polyolefin, etc.) or polyurethane. The spool may then be engaged with the housing at block 230. The first portion of the spool, for example, may be at least partially disposed within the housing, as described above.

In some embodiments, the spool may be engaged with a spring and a retainer at block 240, and the retainer may be attached to the spool to capture a spring between the housing and the retainer at block 250. For example, a spring may be disposed around the first portion of the spool. A retainer configured to be received by a first end of the spool may be provided, and the second end of the spring may be engaged to the housing such that a position of the second end of the spring remains fixed with respect to the housing. Likewise, the first end of the spring may be engaged with the retainer, and the retainer may be attached to the first end of the spool such that the retainer is biased away from the housing, as described in greater detail above.

It will be understood that each block or step of the flowchart illustration of Fig. 11, and combinations of blocks, can be implemented by computer program instructions. These computer program instructions may be loaded onto a computer or other programmable apparatus to produce a machine, such that the instructions which execute on the computer or other programmable apparatus form means for implementing the functions specified in the flowchart block(s) or step(s). These computer program instructions may also be stored in a computer-readable memory that can direct a computer or other programmable apparatus to function in a particular manner, such that the instructions stored in the computer-readable memory may produce an article of manufacture including instruction means which can implement the function specified in the flowchart block(s) or step(s). The computer program instructions may also be loaded onto a computer or other programmable apparatus, among other things, to cause a series of operational steps to be performed on the computer or other programmable apparatus. This may produce a computer implemented process such that the instructions which execute on the computer or other programmable apparatus provide steps for implementing the functions specified in the flowchart block(s) or step(s).

Accordingly, blocks or steps of the flowchart illustration support, among other things, combinations of means for performing the specified functions, combinations of steps for performing the specified functions and program instruction means for performing the specified functions. It will also be understood that each block or step of the flowchart illustration, and combinations thereof, can be implemented by special purpose hardware-based computer systems which perform the specified functions or steps, or combinations of special purpose hardware and computer instructions.

As described above and shown in the associated figures, embodiments of the air/water valve assembly are configured to be disposable, such that the valve assemblies may be removed from an endoscope or other medical equipment and disposed of after use in a single procedure. Accordingly, embodiments of the valve assembly described above do not include numerous precision-machined metal components or multiple separate seals that need to be assembled, nor does the valve assembly require adhesive for attaching machined parts together, which can be complicated and costly. Rather, embodiments of the invention reduce the parts count of the assembly to 5 parts, while still allowing for compatibility of the valve assembly with existing valve bodies. Moreover, embodiments of the valve assembly make use of high volume production processes such as injection molding to minimize manufacturing costs. In addition, the shorter duty life allows for less expensive materials to be used, as the valve assembly does not need to withstand repeated use.

Other modifications and other embodiments of the invention set forth herein will come to mind to one skilled in the art to which this invention pertains having the benefit of the teachings presented in the foregoing descriptions and on the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

Further, throughout the description, where compositions are described as having, including, or comprising specific components, or where processes systems or methods are described as having, including, or comprising specific steps, it is contemplated that compositions or the present invention may also consist essentially of, or consist of, the recited components, and that the processes or methods of the present invention also consist essentially or consist of the recited steps. Further, it should be understood that the order of steps or order of performing certain actions are immaterial so long as the invention remains operable. Moreover, two or more steps or actions may be conducted simultaneously with respect to the invention disclosed herein.

## Claims

1. A valve assembly for a medical instrument, said valve assembly comprising:
a housing (110);
a spool (120) comprising a first end (121), a second end (122), a longitudinal passageway (123) extending between the first end (121) and the second end (122), and first and second portions (124,125) proximate the first and second ends (121,122), respectively, wherein the first portion (124) of the spool (120) is configured to be received at least partially within the housing (110), and wherein an outer surface (126) of the second portion (125) of the spool (120) defines a plurality of positioning features (127a, 127b, 127c, 127d); and
a sealing member (130) comprising a plurality of sealing rings (132, 133, 134, 135),
wherein each sealing ring (132, 133, 134, 135) defines an opening such that an inner circumferential surface (136) of each sealing ring (132, 133, 134, 135) is configured to sealingly engage a corresponding positioning feature (127a, 127b,127c, 127d) of said plurality of positioning features; **characterized in that** the sealing member (130) further comprises a longitudinal support member (131), wherein the plurality of sealing rings (132, 133, 134, 135) extends circumferentially from the support member (131), wherein each sealing ring (132, 133, 134, 135) is spaced from an adjacent sealing ring (132, 133, 134, 135) a distance along a length of the support member (131) that corresponds to a distance between adjacent positioning features (127a, 127b,127c, 127d),
wherein an inner surface (136) of the support member (131) is configured to engage the outer surface (126) of the spool (120).

2. The valve assembly of Claim 1 further comprising:
a spring (140) comprising a first end (141) and a second end (142), wherein the spring (140) is disposed around the first portion (124) of the spool (120), and
a retainer (150) configured to be received by the first end (121) of the spool (120),
wherein the second end (142) of the spring (140) is configured to engage and remain fixed with respect to the housing (110), and the first end (141) of the spring (140) is configured to engage the retainer (150), such that the retainer (150) is biased away from the housing (110).

3. The valve assembly of Claim 2, wherein the housing (110) defines at least one radial extension (111) configured to engage the second end (142) of the spring (140).

4. The valve assembly of Claim 1, wherein at least a portion of the outer surface (126) of the spool (120) defines a longitudinal groove (128) configured to receive the support member (131) of the sealing member (130).

5. The valve assembly of Claim 4, wherein an outer surface (137) of the support member (131) forms a flush surface with the outer surface (126) of the spool (120) when the sealing member (130) is engaged with the spool (120).

6. The valve assembly of Claim 4, wherein the sealing member (130) is overmolded onto the spool (120).

7. The valve assembly of Claim 4, wherein the spool (120) defines a transverse passageway (129) that is substantially perpendicular and intersects with the longitudinal passageway (123), and wherein the transverse passageway (129) is defined through the longitudinal groove (128).

8. The valve assembly of Claim 4, wherein at least one portion of the longitudinal groove (128) is defined by at least one positioning feature (127a, 127b,127c, 127d).

9. The valve assembly of Claim 1, wherein the spool (120) defines a transverse passageway (129) that is substantially perpendicular to and intersects with the longitudinal passageway (123), and wherein the support member (131) of the sealing member (130) defines a longitudinally extending ring (138) configured to engage an opening (170) of the transverse passageway (129) defined in the outer surface (126) of the spool (120).

10. The valve assembly of Claim 1, wherein the sealing member (130) comprises four sealing rings (132, 133, 134, 135).

11. The valve assembly of Claim 1, wherein the housing (110) defines longitudinal extensions (113) configured to engage the medical instrument within which the valve assembly (100) is mounted.

## Patentansprüche

1. Ventilanordnung für ein medizinisches Instrument, wobei die Ventilanordnung umfasst:
ein Gehäuse (110);
eine Spule (120), umfassend ein erstes Ende (121), ein zweites Ende (122), einen Längsdurchgang (123), der sich zwischen dem ersten Ende (121) und dem zweiten Ende (122) erstreckt, und erste und zweite Abschnitte (124, 125), jeweils nahe den ersten und zweiten Enden (121, 122), wobei der erste Abschnitt (124) der Spule (120) dazu ausgelegt ist, mindestens teilweise in dem Gehäuse (110) aufgenommen zu werden, und wobei eine Außenfläche (126) des zweiten Abschnitts (125) der Spule (120) mehrere Positionsmerkmale (127a, 127b, 127c, 127d) definiert; und
ein Dichtungselement (130), umfassend mehrere Dichtungsringe (132, 133, 134, 135),
wobei jeder Dichtungsring (132, 133, 134, 135) eine Öffnung definiert, sodass eine innere Umfangsfläche (136) von jedem Dichtungsring (132, 133, 134, 135) dazu ausgelegt ist, um abdichtend in ein entsprechendes Positionsmerkmal (127a, 127b, 127c, 127d) der mehreren Positionsmerkmale einzugreifen;
**dadurch gekennzeichnet, dass** das Dichtungselement (130) ferner ein längliches Stützelement (131) umfasst, wobei sich die mehreren Dichtungsringe (132, 133, 134, 135) in Umfangsrichtung von dem Stützelement (131) erstrecken, wobei jeder Dichtungsring (132, 133, 134, 135) von jedem benachbarten Dichtungsring (132, 133, 134, 135) in einem Abstand entlang einer Länge des Stützelements (131) beabstandet ist, der einem Abstand zwischen benachbarten Positionsmerkmalen (127a, 127b, 127c, 127d) entspricht,
wobei eine Innenfläche (136) des Stützelements (131) dazu ausgelegt ist, die Außenfläche (126) der Spule (120) zu greifen.

2. Ventilanordnung nach Anspruch 1, ferner umfassend:
eine Feder (140), umfassend ein erstes Ende (141) und ein zweites Ende (142), wobei die Feder (140) um den ersten Abschnitt (124) der Spule (120) angeordnet ist, und
eine Halterung (150), die dazu ausgelegt ist, von dem ersten Ende (121) der Spule (120) aufgenommen zu werden,
wobei das zweite Ende (142) der Feder (140) dazu ausgelegt ist, in Bezug auf das Gehäuse (110) zu greifen und fixiert zu bleiben, und wobei das erste Ende (141) der Feder (140) dazu ausgelegt ist, die Halterung (150) zu greifen, sodass die Halterung (150) von dem Gehäuse (110) weggeneigt ist.

3. Ventilanordnung nach Anspruch 2, wobei das Gehäuse (110) mindestens eine radiale Verlängerung (111) definiert, die dazu ausgelegt ist, das zweite Ende (142) der Feder (140) zu greifen.

4. Ventilanordnung nach Anspruch 1, wobei mindestens ein Abschnitt der Außenfläche (126) der Spule (120) eine Längsnut (128) definiert, die dazu ausgelegt ist, das Stützelement (131) des Dichtungselements (130) aufzunehmen.

5. Ventilanordnung nach Anspruch 4, wobei eine Außenfläche (137) des Stützelements (131) eine ebene Oberfläche mit der Außenfläche (126) der Spule (120) bildet, wenn das Dichtungselement (130) mit der Spule (120) im Eingriff ist.

6. Ventilanordnung nach Anspruch 4, wobei das Dichtungselement (130) auf die Spule (120) augespritzt ist.

7. Ventilanordnung nach Anspruch 4, wobei die Spule (120) einen Querverbindungsgang (129) definiert, der im Wesentlichen senkrecht ist, und der den Längsdurchgang (123) schneidet, und wobei der Querverbindungsgang (129) durch die Längsnut (128) definiert ist.

8. Ventilanordnung nach Anspruch 4, wobei mindestens ein Abschnitt der Längsnut (128) durch mindestens ein Positionsmerkmal (127a, 127b, 127c, 127d) definiert ist.

9. Ventilanordnung nach Anspruch 1, wobei die Spule (120) einen Querverbindungsgang (129) definiert, der im Wesentlichen senkrecht zu dem Längsdurchgang (123) ist und diesen schneidet, und wobei das Stützelement (131) des Dichtungselements (130) einen sich in Längsrichtung erstreckenden Ring (138) definiert, der dazu ausgelegt ist, eine Öffnung (170) des Querverbindungsgangs (129) zu greifen, die in der Außenfläche (126) der Spule (120) definiert ist.

10. Ventilanordnung nach Anspruch 1, wobei das Dichtungselement (130) vier Dichtungsringe (132, 133, 134, 135) umfasst.

11. Ventilanordnung nach Anspruch 1, wobei das Gehäuse (110) längliche Verlängerungen (113) definiert, die dazu ausgelegt sind, das medizinische Instrument, in dem die Ventilanordnung (100) angebracht ist, zu greifen.

## Revendications

1. Un ensemble formant vanne pour un instrument médical, ledit ensemble formant vanne comprenant :
un boîtier (110) ;
un tournant (120) comprenant une première extrémité (121), une deuxième extrémité (122), un passage longitudinal (123) s'étendant entre la première extrémité (121) et la deuxième extrémité (122), et des première et deuxième parties (124, 125) proches respectivement des première et deuxième extrémités (121, 122), la première partie (124) du tournant (120) étant configurée pour être reçue au moins partiellement à l'intérieur du boîtier (110), et une surface extérieure (126) de la deuxième partie (125) du tournant (120) définissant une pluralité de caractéristiques de positionnement (127a, 127b, 127c, 127d) ; et
un organe d'étanchéité (130) comprenant une pluralité de bagues d'étanchéité (132, 133, 134, 135),
chaque bague d'étanchéité (132, 133, 134, 135) définissant une ouverture telle qu'une surface circonférentielle intérieure (136) de chaque bague d'étanchéité (132, 133, 134, 135) est configurée pour venir en engagement, avec étanchéité, avec une caractéristique de positionnement correspondante (127a, 127b, 127c, 127d) de ladite pluralité des caractéristiques de positionnement ;
**caractérisé en ce que** l'organe d'étanchéité (130) comprend en outre un élément de support longitudinal (131), la pluralité de bagues d'étanchéité (132, 133, 134, 135) s'étendant circonférentiellement depuis l'élément de support (131), chaque bague d'étanchéité (132, 133, 134, 135) étant séparée d'une bague d'étanchéité (132, 133, 134, 135) adjacente d'une distance selon une longueur de l'élément de support (131) qui correspond à une distance entre des caractéristiques de positionnement (127a, 127b, 127c, 127d) adjacentes,
une surface interne (136) de l'élément de support (131) étant configurée pour venir en engagement avec la surface externe (126) du tournant (120).

2. L'ensemble formant vanne selon la revendication 1 comprenant en outre :
un ressort (140) comprenant une première extrémité (141) et une deuxième extrémité (142), le ressort (140) étant disposé autour de la première partie (124) du tournant (120), et
un organe de retenue (150) configuré pour être reçu par la première extrémité (121) du tournant (120),
la deuxième extrémité (142) du ressort (140) étant configurée pour venir en engagement avec le boîtier (110) et rester fixe par rapport à celui-ci, et la première extrémité (141) du ressort (140) étant configurée pour venir en engagement dans l'organe de retenue (150), de sorte que l'organe de retenue (150) soit sollicité en écartement du boîtier (110).

3. L'ensemble formant vanne selon la revendication 2, dans lequel le boîtier (110) définit au moins une extension radiale (111) configurée pour venir en engagement avec la deuxième extrémité (142) du ressort (140).

4. L'ensemble formant vanne selon la revendication 1, dans lequel au moins une partie de la surface extérieure (126) du tournant (120) définit une rainure longitudinale (128) configurée pour recevoir l'élément support (131) de l'organe d'étanchéité (130).

5. L'ensemble formant vanne selon la revendication 4, dans lequel une surface extérieure (137) de l'élément de support (131) forme une surface en affleurement avec la surface extérieure (126) du tournant (120) lorsque l'organe d'étanchéité (130) est en engagement avec le tournant (120).

6. L'ensemble formant vanne selon la revendication 4, dans lequel l'organe d'étanchéité (130) est surmoulé sur le tournant (120).

7. L'ensemble formant vanne selon la revendication 4, dans lequel le tournant (120) définit un passage transversal (129) qui est sensiblement perpendiculaire au passage longitudinal (123) et vient en intersection avec ce dernier, et dans lequel le passage transversal (129) est défini au travers de la rainure longitudinale (128).

8. L'ensemble formant vanne selon la revendication 4, dans lequel au moins une partie de la rainure longitudinale (128) est définie par au moins une caractéristique de positionnement (127a, 127b, 127c, 127d).

9. L'ensemble formant vanne selon la revendication 1, dans lequel le tournant (120) définit un passage transversal (129) qui est sensiblement perpendiculaire au passage longitudinal (123) et vient en intersection avec celui-ci, et dans lequel l'élément de support (131) de l'organe d'étanchéité (130) définit un anneau (138) s'étendant longitudinalement, configuré pour venir en engagement avec une ouverture (170) du passage transversal (129) défini dans la surface externe (126) du tournant (120).

10. L'ensemble formant vanne selon la revendication 1, dans lequel l'organe d'étanchéité (130) comprend quatre bagues d'étanchéité (132, 133, 134, 135).

11. L'ensemble formant vanne selon la revendication 1, dans lequel le boîtier (110) définit des extensions longitudinales (113) configurées pour venir en engagement avec l'instrument médical dans lequel l'ensemble formant vanne (100) est monté.
